# EUROPÄISCHE PATENTANMELDUNG

(11) **EP 4 414 400 A1**
(43) Veröffentlichungstag der Anmeldung: **14.08.2024**
(21) Anmeldenummer: 23155763.8
(22) Anmeldetag: 09.02.2023
(51) Int. Cl.: C08G 18/79, C08G 18/02, C08G 18/20, C07D 295/037, B01J 27/12, B01J 31/02, C08G 18/78

(54) **POLYISOCYANATE MIT VERBESSERTEN EIGENSCHAFTEN**

(71) Anmelder: Covestro Deutschland AG, 51373 Leverkusen (DE)
(72) Erfinder: Richter, Frank, 51373 Leverkusen (DE)
(74) Vertreter: Levpat

(57) **Zusammenfassung**

Die Erfindung betrifft ein Verfahren zur Herstellung modifizierter Isocyanate, bei dem mindestens ein organisches Di- und/oder Triisocyanat in zumindest zeitweiliger Anwesenheit einer Komponente A1 umfassend mindestens ein Tetramethylammoniumsalz und/oder mindestens ein cyclisches Ammoniumsalz mit einem Kation der Formel I, 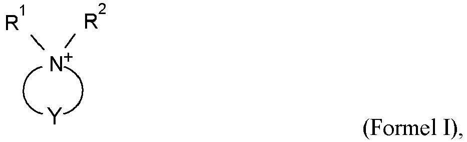 wobei
Y für ein, ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
die N-ständigen Substituenten R¹ und R² untereinander ein Ringsegment X bilden, für das die gleiche oder verschiedene vorstehend für Y gegebene Definition gilt, umgesetzt wird,
die Umsetzung bei Erreichen eines vorgebbaren Umsetzungsgrades, bezogen auf die Gesamtmenge an NCO-Gruppen des mindestens einen organischen Di- und/oder Triisocyanates, durch Zugabe einer unterstöchiometrischen Menge, bezogen auf die molare Menge des Kations der Formel I in Komponente A1, einer Komponente A2, umfassend mindestens eine saure Verbindung, die einen pKₛ-Wert unterhalb 4,0 aufweist und verschieden von HF ist, gestoppt wird
und das erhaltene modifizierte Isocyanat nach optionaler Reinigung mit einer Komponente A3, umfassend mindestens eine saure Verbindung, die einen pKₛ-Wert unterhalb 4,0 aufweist und verschieden von HF ist, versetzt wird,
wobei die mindestens eine saure Verbindung in Komponente A2 und A3 verschieden voneinander oder gleich sein können.

## Beschreibung

Die Erfindung betrifft Polyisocyanate mit verbesserten Eigenschaften wie Geruchsfreiheit, extrem niedrigem Monomerengehalt, guter Monomerenstablität und geringer Trübungsneigung. Außerdem betrifft die Erfindung ein Verfahren zur Herstellung dieser Polyisocyanate mit verbesserten Eigenschaften selbst und deren Verwendung zur Herstellung von Polyurethankörpern oder Beschichtungen sowie die Polyurethankörper oder Beschichtungen. Weiterhin betrifft die Erfindung Ein- oder Zwei-Komponenten-Systeme umfassend die Polyisocyanate mit verbesserten Eigenschaften.

Die Oligo- bzw. Polymerisierung von Isocyanaten insbesondere unter Bildung von höhermolekularen Oligomerengemischen mit Uretdion- ("Dimer"), Isocyanurat- ("Trimer") und/oder Iminooxadiazindionstrukturen ("asymmetrisches Trimer") im Molekülgerüst ist seit langem bekannt. Wie vorstehend erkennbar, liegen der Oligo- bzw. Polymerisierung von Isocyanaten prinzipiell die gleichen chemischen Reaktionen zugrunde. Die Umsetzung einer kleineren Anzahl von Isocyanaten miteinander bezeichnet man als Oligomerisierung. Die Umsetzung einer größeren Anzahl von Isocyanaten bezeichnet man als Polymerisierung. Im Rahmen der vorliegenden Erfindung wird die vorstehend beschriebene Oligomerisierung bzw. Polymerisierung von Isocyanaten zusammenfassend als Isocyanatmodifizierung oder Modifizierung von Isocyanaten bezeichnet.

Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert) und das erhaltene Polyisocyanat in der Regel vom nicht umgesetzten Monomer abtrennt. Eine Zusammenstellung dieser Verfahren des Standes der Technik findet sich in H. J. Laas et al., J. Prakt. Chem. 1994, 336, 185 ff.

Als Modifizierungskatalysatoren haben sich ionisch aufgebaute Verbindungen bewährt, da sie in sehr geringer Menge, relativ zum umzusetzenden Monomer, eingesetzt werden können und äußerst schnell zum gewünschten Ergebnis führen, wobei die Kationen insbes. unter dem Aspekt der Löslichkeit des jeweiligen Salzes im Isocyanatmilieu von Bedeutung sind.

WO 2015/124504 A1 und WO 2017/029266 A1 beschreiben sehr stabile Ammoniumsalze, bei denen das ladungstragende Stickstoffatom Teil eines Ringsystems ist. Diese Verbindungen weisen allerdings den Nachteil auf, im isocyanatfunktionellen Polyisocyanatharz schlecht löslich zu sein und daher Trübungen in den finalen Verfahrensprodukten verursachen zu können.

Nach der Lehre der WO 2021/122508 A1 wird dieses Problem durch den Einbau von Hydroxifunktionen in das Katalysatormolekül umgangen. Ein Nachteil ist es jedoch, dass die speziellen, hydroxi-funktionellen Ammoniumsalze kommerziell nicht verfügbar sind und ihre Synthese sehr aufwändig ist. Wohlfeile, hydroxi-funktionelle Ammoniumsalze in denen die Hydroxifunktion nicht an ein C-Atom des Ladungs-tragenden, das Stickstoffatom enthaltenden Cyclus' gebunden ist, weisen keinerlei Vorteile im Sinne der vorliegenden Erfindung auf: sie sind weder Geruchs-frei noch sind die im Verfahren anfallenden und typischerweise recyclierten Monomeren frei von störenden Katalysator-Zersetzungsprodukten.

Der Erfindung lag daher die Aufgabe zugrunde, Polyisocyanate mit verbesserten Eigenschaften wie Geruchsfreiheit, extrem niedrigem Monomerengehalt von < 0,1 Gew.-%, hohe Rückspaltstabilität und geringe Trübungsneigung zur Verfügung zu stellen. Insbesondere bestand die Aufgabe, Polyisocyanate mit einem hohen Anteil an Iminooxadiazindionstrukturen und vorstehend genannten verbesserten Eigenschaften zur Verfügung zu stellen.

Diesem Bedarf Rechnung tragend betrifft die Erfindung in einem ersten Gegenstand ein Verfahren zur Herstellung modifizierter Isocyanate, bei dem mindestens ein organisches Di- und/oder Triisocyanat in zumindest zeitweiliger Anwesenheit einer Komponente A1 umfassend mindestens ein Tetramethylammoniumsalz und/oder mindestens ein cyclisches Ammoniumsalz mit einem Kation der Formel I, 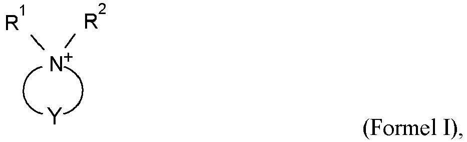 wobei
Y für ein, ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
die N-ständigen Substituenten R¹ und R² untereinander ein Ringsegment X bilden, für das die gleiche oder verschiedene vorstehend für Y gegebene Definition gilt, umgesetzt wird, die Umsetzung bei Erreichen eines vorgebbaren Umsetzungsgrades bezogen auf die Gesamtmenge an NCO-Gruppen des mindestens einen organischen Di- und/oder Triisocyanates durch Zugabe einer unterstöchiometrischen Menge, bezogen auf die molare Menge des Kations der Formel I in Komponente A1, einer Komponente A2, umfassend mindestens eine saure Verbindung, die einen pKₛ-Wert unterhalb 4,0 aufweist und verschieden von HF ist, gestoppt wird und das erhaltene modifizierte Isocyanat (optional nach weiterer Reinigung) mit einer Komponente A3, umfassend mindestens eine saure Verbindung, die einen pKₛ-Wert unterhalb 4,0 aufweist und verschieden von HF ist, versetzt wird, wobei die mindestens eine saure Verbindung in Komponente A2 und A3 verschieden voneinander oder gleich sein können.

Ebenfalls diesem Bedarf Rechnung tragend, betrifft die Erfindung in einem Gegenstand ein Katalysator-Kit für die Isocyanatmodifizierung, umfassend drei separate Komponenten A1, A2 und A3, wobei
a) die Komponente A1 mindestens ein Tetramethylammoniumsalz und/oder mindestens ein cyclisches Ammoniumsalz mit einem Kation der Formel I, 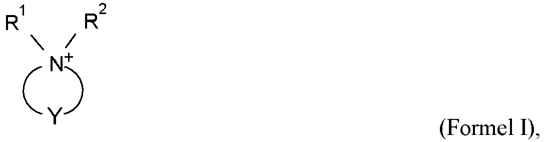 enthält, wobei
   Y für ein, ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
   die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
   die N-ständigen Substituenten R¹ und R² untereinander ein Ringsegment X bilden, für das die gleiche oder verschiedene vorstehend für Y gegebene Definition gilt;
b) die Komponente A2 in einer unterstöchiometrischen Menge, bezogen auf die molare Menge des Kations der Formel I in Komponente A1, eingesetzt wird und mindestens eine saure Verbindung umfasst oder daraus besteht, die einen pKₛ-Wert unterhalb 4,0 aufweist und verschieden von HF ist;
c) die Komponente A3 mindestens eine saure Verbindung enthält, die einen pKₛ-Wert unterhalb 4,0 aufweist und verschieden von HF ist, wobei die mindestens eine saure Verbindung in Komponente A2 und A3 verschieden voneinander oder gleich sein können.

Ebenfalls diesem Bedarf Rechnung tragend betrifft die Erfindung in einem Gegenstand eine Verwendung mindestens eines erfindungsgemäßen Katalysator-Kits zur Modifizierung von Isocyanaten und zur Verhinderung von Trübungen in den modifizierten Isocyanaten.

Bevorzugt bedeuten die Bezugnahmen auf "umfassend", "enthaltend" usw. "im Wesentlichen bestehend aus" und ganz besonders bevorzugt "bestehend aus". Die in den Patentansprüchen und in der Beschreibung genannten weiteren Ausführungsformen können beliebig, insbesondere auch unter den verschiedenen erfindungsgemäßen Gegenständen, kombiniert werden, sofern sich aus dem Kontext nicht eindeutig das Gegenteil ergibt.

"Mindestens ein", wie hierin verwendet, bezieht sich auf 1 oder mehr, beispielsweise 2, 3, 4, 5, 6, 7, 8, 9 oder mehr. Im Zusammenhang mit Bestandteilen der hierin beschriebenen Verbindungen bezieht sich diese Angabe nicht auf die absolute Menge an Molekülen, sondern auf die Art des Bestandteils. "mindestens ein cyclisches Ammoniumsalz" bedeutet daher beispielsweise, dass nur eine Art von cyclischem Ammoniumsalz oder mehrere verschiedene Arten von cyclischen Ammoniumsalzen, ohne Angaben über die Menge der einzelnen Verbindungen zu machen, enthalten sein können.

Zahlenwerte, die hierin ohne Dezimalstellen angegeben sind, beziehen sich jeweils auf den vollen angegebenen Wert mit einer Dezimalstelle. So steht beispielsweise "99 %" für "99,0 %".

Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Vorliegend zählt bei mehrbasigen Säuren der niedrigste Wert als pK_{S}-Wert, beispielsweise bei Phosphorsäure ist es der pKs1 von 2,16 und nicht die weiteren Werte pKs2 = 7,20 und pKs3 = 12,33.

Vorliegend ist der Begriff "aliphatisch" definiert als nicht-aromatische Kohlenwasserstoffgruppen, die gesättigt oder ungesättigt sind.

Vorliegend ist der Begriff "araliphatisch" definiert als Kohlenwasserstoffreste, die sowohl aus einer aromatischen als auch aus einer gesättigten oder ungesättigten Kohlenwasserstoffgruppe bestehen, die direkt an den aromatischen Rest gebunden ist.

Vorliegend ist der Begriff "alicyclisch" oder "cycloaliphatisch" definiert als gegebenenfalls substituierte, carbocyclische oder heterocyclische Verbindungen oder Einheiten, die nicht aromatisch sind (wie z.B. Cycloalkane, Cycloalkene oder Oxa-, Thia-, Aza- oder Thiazacycloalkane). Besondere Beispiele sind Cyclohexylgruppen, Cyclopentylgruppen und ihre N- oder O-heterocyclischen Derivate wie z.B. Pyrimidin, Pyrazin, Tetrahydropyran oder Tetrahydrofuran.

Für den Fall, dass die Gruppen oder Verbindungen als "gegebenenfalls substituiert" oder "substituiert" offenbart werden, sind geeignete Substituenten -F, -Cl, -Br, -I, -OCH3, OCH2CH3, - O-Isopropyl oder -O-n-Propyl, -OCF3, -CF3, -S-C1-6-Alkyl und/oder (gegebenenfalls über ein angehängtes Heteroatom) eine lineare oder verzweigte, aliphatische und/oder alicyclische Struktureinheit mit 1 bis 12 Kohlenstoffatomen, die jeweils als Ersatz für ein kohlenstoffgebundenes Wasserstoffatom des betreffenden Moleküls fungiert. Bevorzugte Substituenten sind Halogen (insbesondere -F, -Cl), C₁-C₆-Alkoxy (insbesondere Methoxy und Ethoxy), Trifluormethyl und Trifluormethoxy, die jeweils als Ersatz für ein an Kohlenstoff gebundenes Wasserstoffatom des betreffenden Moleküls fungieren.

In der Komponente A1 ist erfindungsgemäß mindestens ein Tetramethylammoniumsalz und/oder mindestens ein cyclisches Ammoniumsalz mit einem Kation der Formel I enthalten, wobei das cyclische Ammoniumsalz mit einem Kation der Formel I bevorzugt ist.

In einer ersten bevorzugten Ausführungsform steht Y für ein gemeinsam mit dem ladungstragenden Stickstoffatom vier- bis sieben-gliedriges, ggf. weitere Substituenten tragendes Alkylenkettensegment.

In einer ebenfalls bevorzugten Ausführungsform stehen R¹ und R² gemeinsam mit dem ladungstragenden Stickstoffatom für ein zu Y gleiches oder von Y verschiedenes Ringsegment X, wobei X ein ggf. weitere Substituenten tragendes C₄-C₆-Alkylenkettensegment ist und optional weitere Substituenten tragen kann.

In einer weiteren bevorzugten Ausführungsform sind das Segment Y und/oder das Ringsegment X linear aufgebaut.

Kationen der Formel I in denen die N-ständigen Substituenten R¹ und R² untereinander ein Ringsystem bzw. das Ringsegment X bilden, stellen spirocyclische Verbindungen dar. Letztere sind in einfacher Weise durch Umsetzung von sekundären Aminen in denen das Stickstoffatom Teil eines Ringsystems ist, mit geeignet substituierten Dihalogenalkanen und anschließendem Anionenaustausch zugänglich. Ein bevorzugter Weg zu ihrer effizienten Synthese ist Beispiel 1 zu entnehmen.

Bevorzugt können X und Y in Formel I unabhängig voneinander für ggf. substituierte Alkylengruppen stehen, wobei C₄-C₆- Alkylenketten insbesondere in beiden N-zentrierten Ringen bevorzugt sind. Die C₄-C₆- Alkylenketten sind dabei vorzugsweise linear aufgebaut. Diese sind z.B. in einfacher Weise durch Reaktion von ggf. C-substituierten Pyrrolidinen, Piperidinen und Azepanen (1H-Hexahydroazepinen) mit, ggf. substituierten, 1,4-, 1,5- sowie 1,6-Dihalogenalkanen zugänglich, wobei Halogen für Cl, Br und I, bevorzugt Cl, steht.

Weiterhin sind beispielsweise durch analoge Umsetzung von ggf. C-substituierten Oxazolidinen, Isoxazolidinen, Oxazinanen, Morpholinen und Oxazepanen sowie den Analoga der vorstehend genannten N-O-Heterocyclen, die S statt O enthalten, weiterhin Imidazolidinen, Pyrazolidinen, Piperazinen und strukturell verwandten Verbindungen mit o.g. Dihalogenalkanen auch Vertreter zugänglich, die in einem der Segmente X oder Y der allg. Formel I durch Heteroatome unterbrochene C-Ketten aufweisen. Bei den 2 oder mehr Stickstoffatome enthaltenden Spezies besteht darüber hinaus die Möglichkeit, durch entsprechende Variation der Reaktionsbedingungen auch Salze mit zwei- bzw. mehrfach geladenem Kation zu erzeugen oder durch vorherige geeignete Substitution des bzw. der N-Atome zu einfach positiv geladenen Kationen der Formel I zu gelangen in denen sich ein bzw. mehrere exocyclische(r) Alkylsubstituent(en) an dem bzw. den dreibindigen N-Atom(en) des Ringes X bzw. Y befindet bzw. befinden.

Natürlich lässt sich auch durch geeignete Wahl des Alkylierungsmittels eine strukturelle Variation in das Ringsegment X oder Y einbringen, beispielhaft genannt seien hierfür Reaktionen von alphaomega-Dihalogenalkylethern mit den o.g. sekundären Aminen.

In einer weiteren bevorzugten Ausführungsform stehen R¹ und R² unabhängig voneinander für gleiche oder verschiedene C₁-C₈-Alkylsubstituenten oder für gleiche oder verschiedene, ggf. am aromatischen Kern substituierte Benzyl-Reste, bevorzugt für gleiche oder verschiedene C₁-C₆-Alkylsubstituenten und besonders bevorzugt für gleiche oder verschiedene, linear aufgebaute C₁-C₆-Alkylsubstituenten und ganz besonders bevorzugt für Methyl stehen.

Als Anionen können bei den Verbindungen der Formel I prinzipiell alle Strukturtypen zum Einsatz kommen, die als katalytisch aktiv gegenüber Isocyanaten bekannt sind, bevorzugt sind das Hydroxid, Alkanoat, Carboxylat, Heterocyclen mit mindestens einem negativ geladenen Stickstoffatom im Ring, insbesondere Azolat, Imidazolat, Triazolat, Tetrazolat, Fluorid, Hydrogendifluorid, höhere Polyfluoride oder Mischungen von diesen (Addukte von mehr als einem Äquivalent HF an Fluorid-Ionen enthaltenden Verbindungen), wobei die Fluoride, Hydrogendifluoride und höheren Polyfluoride erfindungsgemäß zu Produkten mit hohem Iminooxadiazindiongruppengehalt führen.

Die erfindungsgemäß einzusetzenden Katalysatoren der Komponente A1 können einzelnen oder in beliebigen Mischungen untereinander verwendet werden. So liegen die Lösungen quaternärer Ammoniumhydroxide in verschiedenen Alkoholen je nach pKs-Wert der Base und des verwendeten Alkohols teilweise oder vollständig als Ammoniumsalze mit Alkoholat-Anion vor. Dieses Gleichgewicht kann durch Entfernung des aus dieser Reaktion resultierenden Reaktionswassers ganz auf die Seite vollständiger Alkoholatbildung verschoben werden.

Bei dem erfindungsgemäßen Verfahren kann weiter vorgesehen sein, dass die Oligomerisierung unter Anwesenheit eines Lösungsmittels durchgeführt wird.

Zur Durchführung des erfindungsgemäßen Verfahrens können prinzipiell alle bekannten Mono-, Di- oder Polyisocyanate des Standes der Technik einzeln oder in beliebigen Mischungen untereinander eingesetzt werden.

Beispielhaft seien genannt: Pentamethylendiisocyanat (PDI), Hexamethylendiisocyanat (HDI), 2-Methylpentan-1,5-diisocyanat (MPDI), 2,4,4-Trimethyl-1,6-hexandiisocyanat und 2,2,4-Trimethyl-1,6-hexandiisocyanat (TMDI), 4-Isocyanatomethyl-1,8-octandiisocyanat (Nonantriisocyanat, NTI), 3(4)-Isocyanatomethyl-1-methylcyclohexylisocyanat (IMCI), Isophorondiisocyanat (IPDI), 1,3-sowie 1,4-Bis(isocyanatomethyl)benzen (XDI), 1,3- sowie 1,4-Bis(isocyanatomethyl)cyclohexan (H₆XDI), Norbornandiisocyanat (NBDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Polymer-MDI).

Bevorzugt sind aromatische Diisocyanate, d.h. Diisocyanate bei denen beide NCO-Gruppen an ein sp²-hybridisiertes Kohlenstoffatom gebunden sind, oder aliphatische Diisocyanate, d.h. Diisocyanate bei denen beide NCO-Gruppen an ein sp³-hybridisiertes Kohlenstoffatom gebunden sind.

Besonders bevorzugt sind PDI, HDI, MPDI, TMDI, NTI, IPDI, IMCI, XDI, H₆XDI, MDI, oder NBDI und ganz besonders bevorzugt sind Pentamethylendiisocyanat (PDI), Hexamethylendiisocyanat (HDI), Isophorondiisocyanat (IPDI), 1,3- sowie 1,4-Bis(isocyanatomethyl)cyclohexan (H₆XDI) und/oder Norbomandiisocyanat (NBDI).

Es ist belanglos, nach welchen Verfahren die vorstehend genannten Isocyanate generiert werden, d.h. mit oder ohne Verwendung von Phosgen.

Die Menge des im erfindungsgemäßen Verfahren einzusetzenden Katalysators der Komponente A1 richtet sich in erster Linie nach dem verwendeten organischen Isocyanat und der angestrebten Reaktionsgeschwindigkeit und liegt vorzugsweise zwischen ≥ 0,001 und ≤ 5 mol-%, bevorzugt zwischen ≥ 0,002 und ≤ 2 mol-%, bezogen auf die Summe der Stoffmengen des eingesetzten Isocyanates und des Katalysators.

Der Katalysator kann im erfindungsgemäßen Verfahren oder im erfindungsgemäßen Katalysator-Kit unverdünnt oder in Lösungsmitteln gelöst eingesetzt werden. Als Lösungsmittel kommen dabei alle Verbindungen in Frage, die nicht mit dem Katalysator reagieren und ihn in ausreichendem Maße zu lösen vermögen, z.B. ggf. halogenierte, aliphatische oder aromatische Kohlenwasserstoffe, Alkohole, Ketone, Ester sowie Ether. Bevorzugt werden Alkohole verwendet.

Das erfindungsgemäße Verfahren kann im Temperaturbereich von 0 °C bis + 250 °C, bevorzugt 20 °C bis 200 °C, besonders bevorzugt 40 °C bis 150 °C erfolgen und bei beliebigen Umsetzungsgraden, bevorzugt nachdem 5 bis 80 %, besonders bevorzugt 10 bis 60 %, des eingesetzten Isocyanates umgesetzt wurden, unterbrochen werden.

Zum Stoppen der Umsetzung (im Folgenden auch Desaktivierung des Katalysators oder Terminierung der katalysierten Isocyanatoligomerisierung genannt) können beliebige Methoden verwendet werden, wobei es erfindungswesentlich ist, dass eine chemische Desaktivierung des Katalysators in mindestens zwei Schritten erfolgt, um zu den erfindungsgemäßen Produkten mit niedriger Kristallisationsneigung und niedrigem Restmonomergehalt zu gelangen.

Erfindungsgemäß wird daher zur Terminierung der katalysierten Isocyanatoligomerisierung eine zum eingesetzten Katalysator unterstöchiometrische aber für die Terminierung der katalysierten Reaktion ausreichende Menge an mindestens einem Katalysatorgift (im weiteren Text auch als "Stopper" und gesammelt als Komponente A2 bezeichnet) eingesetzt und eine zweite Dosis als Komponente A3 des gleichen oder eines anderen Katalysatorgiftes nach der, bevorzugt destillativen, Monomerabtrennung in das erfindungsgemäße Produkt mit niedrigem Restmonomergehalt dosiert. Die als zweite Dosis zugegebene Menge der Komponente A3 (im weiteren Text auch als "Stabilisator" bezeichnet) kann über einen breiten Bereich frei gewählt werden, bevorzugt wird sie in einer molaren Menge zugesetzt, die stöchiometrisch, besonders bevorzugt überstöchiometrisch, bezogen auf die rechnerisch nach Abzug der, bereits als Komponente A2 anwesenden Menge verbliebenen, molaren Menge des Kations der Formel I in Komponente A1 ist. Hierbei ist "überstöchiometrisch" bevorzugt so zu verstehen, dass nicht mehr als die doppelt-molare Menge Katalysatorgift zur molaren Menge des Kations der Formel I ins Komponente A1 eingesetzt wird.

Erst durch diese erfindungsgemäße Maßnahme wird sichergestellt, auch für lange Zeit und auch bei niedriger Temperatur und/oder bei Einsatz von typischen Lacklösemitteln für das Polyisocyanatharz geruchsneutrale, kristallisationsstabile Produkte mit extrem niedrigen Monomergehalt und hoher Rückspaltstabilität zu generieren. Weiterhin profitiert man nach dem erfindungsgemäßen Verfahren von einer signifikant verbesserten Prozessstabilität durch einheitlichere Katalysatorverbräuche ohne Drifts und eine geringere Neigung zur Bildung von Anbackungen, Trübungen, Feststoffabscheidungen etc. in den Anlagen.

Als Stopper geeignet sind generell saure Verbindungen, welche die im vorliegenden Text definierten pKs-Werte aufweisen und verschieden von HF sind, beispielsweise Alkan- sowie Aryl-sulfonsäuren wie z.B. Napthalen-mono- sowie disulfonsäuren, Toluolsulfonsäure Dodecylbenzolsulfonsäure, Methansulfonsäure, Phosphorsäure und saure Ester der Phosphorsäure, wie beispielsweise Dibutylphosphat und/oder Monobutylphosphat sowie beliebige Mischungen der vorstehend genannten Verbindungen.

In einer bevorzugten Ausführungsform ist die mindestens eine saure Verbindung der Komponente A2 und/oder ausgewählt ist aus der Gruppe umfassend oder bestehend aus Alkan- sowie Aryl-sulfonsäuren wie z.B. Napthalen-mono- sowie disulfonsäuren, Toluolsulfonsäure Dodecylbenzolsulfonsäure, Methansulfonsäure, Phosphorsäure und saure Ester der Phosphorsäure, wie beispielsweise Dibutylphosphat und/oder Monobutylphosphat sowie beliebige Mischungen der vorstehend genannten Verbindungen, bevorzugt aus aromatischen Sulfonsäuren und besonders bevorzugt aus Dodecylbenzolsulfonsäure und Toluolsulfonsäure.

In einer weiteren bevorzugten Ausführungsform wird nicht umgesetztes organisches Isocyanat nach Desaktivierung des Katalysatorsystems nach einem beliebigen Verfahren des Standes der Technik, z.B. durch (Dünnschicht)destillation oder Extraktion, abgetrennt und bevorzugt anschließend wiederverwendet (recycliert).

Ganz allgemein sind die erfindungsgemäßen, cyclische Segmente aufweisenden Katalysatoren unabhängig vom Anion, das für die katalytische Aktivität und Selektivität verantwortlich ist, im umzusetzenden organischen Isocyanat wesentlich stabiler als die literaturbekannten offenkettigen Derivate des Standes der Technik was allerdings zu Nachteilen bei der technischen Durchführung infolge schlechter Löslichkeit und Trübungsbildung der Katalysatorfolgeprodukte im Polyisocyanat führen kann aber andererseits Vorteile durch die geringere Kontamination des Recyclat-Monomers durch N-haltige Spaltprodukte aus der Zersetzung des Kations nach sich zieht.

Nach einer besonderen, kontinuierlich arbeitenden Ausführungsform des erfindungsgemäßen Verfahrens kann die Oligomerisierung kontinuierlich, z.B. in einem Rohrreaktor, vorgenommen werden.

Mit dem erfindungsgemäßen Modifizierverfahren ist ganz allgemein eine breite Palette qualitativ hochwertiger und deshalb für den Polyurethansektor sehr wertvoller modifizierter Isocyanate zugänglich. Ein weiterer Gegenstand der vorliegenden Erfindung ist ein modifiziertes Isocyanat, erhältlich oder hergestellt durch das erfindungsgemäße Verfahren.

Abhängig vom verwendeten Ausgangs(di)isocyanat und den Reaktionsbedingungen entstehen beim erfindungsgemäßen Verfahren Polyisocyanate vom sog. Isocyanat-Trimertyp (d.h. enthaltend Isocyanurat- und/oder Iminooxadiazindionstrukturen) mit geringem Anteil an Uretdiongruppen ("Isocyanat-Dimere"). Bei steigender Reaktionstemperatur steigt der Anteil letzterer in den Verfahrensprodukten in der Regel an.

Bevorzugt sind hoch-Iminooxadiazindiongruppen-haltige Polyisocyanate die erfindungsgemäß durch Polyfluoridkatalyse zugänglich sind. Hierbei ist der Begriff "hoch-Iminooxadiazindiongruppen-haltig" zu verstehen als mindestens 30 mol-%, bevorzugt >35 mol-% und besonders bevorzugt >40 mol-% bezogen auf Summe an Isocyanurat- und Iminooxadiazindiongruppen. Die vorstehend genannten molaren Verhältnisse lassen sich beispielsweise NMR-spektroskopisch ermitteln (s. Beispielteil).

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Daher ist die Verwendung der erfindungsgemäßen modifizierten Isocyanate zur Herstellung von geschäumten oder ungeschäumten Kunststoffen sowie Lacken, Beschichtungsmitteln, Klebstoffen und Zuschlagstoffen ein weiterer Gegenstand der vorliegenden Erfindung. Folglich sind Polyurethankörper, erhältlich oder hergestellt durch Umsetzung wenigstens eines monomeren Diisocyanats und/oder Polyisocyanats mit wenigstens einer Polyolkomponente in Gegenwart der erfindungsgemäßen Katalysatorkomponente ein weiterer Gegenstand der Erfindung. Für den Fall, dass es sich um geschäumte Polyurethankörper handelt, sind es bevorzugt PIR-Schäume.

Die erfindungsgemäßen Verfahrensprodukte können als solche oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

Weitere Gegenstände der vorliegenden Erfindung sind ein Ein- oder Zweikomponenten-Systeme, enthaltend eine Komponente A), umfassend mindestens ein erfindungsgemäßes modifiziertes Isocyanat, und eine Komponente B), umfassend mindestens eine NCO-reaktive Verbindung, sowie eine Beschichtung, erhältlich oder hergestellt durch, ggf. unter Wärmeeinwirkung und/oder in Anwesenheit eines Katalysators, Härten eines erfindungsgemäßen Ein- oder Zwei-Komponenten-Systems aber auch die mit mindestens einem erfindungsgemäßen, gegebenenfalls unter Wärmeeinwirkung ausgehärteten, Ein- oder Zweikomponenten-System, beschichteten Substrate.

Bereits aus der überraschenden Beobachtung, dass die erfindungsgemäßen modifizierten Isocyanate keine verzögert einsetzende Trübung aufweisen, wird deutlich, dass die erfindungsgemäßen Produkte vom Stand der Technik strukturell und in der Zusammensetzung verschieden sein müssen. Die erfindungsgemäßen modifizierten Isocyanate unterscheiden sich über die Kationen (aus Komponente A1) zu Anionen (aus Komponenten A2 und A3) Verhältnisse, genauer das Kationen (aus 1) zu Halogenid-Verhältnis, bevorzugt hierbei das Kationen (aus A1) zu Chlorid und/oder Kationen (aus A1) zu Bromid Verhältnis, sowie das Kationen (aus A1) zu Anionen (aus A2 und/oder A3) Sulfonaten Verhältnis. Insbesondere das Kationen (aus A1) zu Anionen (aus A2 und/oder A3) Verhältnis, wobei die aus A2 und/oder A3 resultierenden Anionen Phosphor und/oder Schwefel, bevorzugt Schwefel enthalten. Die Unterschiede finden sich in den gehärteten oder geschäumten Polyurethankörpern wieder, somit ist ein Verbundbauteil, umfassend einen Werkstoff, der mit einem erfindungsgemäßen Polyurethankörper oder einer erfindungsgemäßen Beschichtung zumindest anteilig verbunden ist, ebenfalls Gegenstand der Erfindung.

Vorliegend hat der Begriff "modifiziertes Isocyanat" die eingangs definierte Bedeutung und steht vorzugsweise für ein Polyisocyanat, welches im statistischen Mittel mindestens 1,5 NCO-Gruppen aufweist. Das erfindungsgemäße modifizierte Isocyanat ist gleichbedeutend mit einer modifizierten Isocyanatzusammensetzung, da z.B. die Kationen und Anionen nicht abtrennbar enthalten sind.

Die vorliegende Erfindung betrifft insbesondere die folgenden Ausführungsformen:
Nach einer ersten Ausführungsform betrifft die Erfindung ein Verfahren zur Herstellung modifizierter Isocyanate, bei dem mindestens ein organisches Di- und/oder Triisocyanat in zumindest zeitweiliger Anwesenheit einer Komponente A1 umfassend mindestens ein Tetramethylammoniumsalz und/oder mindestens ein cyclisches Ammoniumsalz mit einem Kation der Formel I, 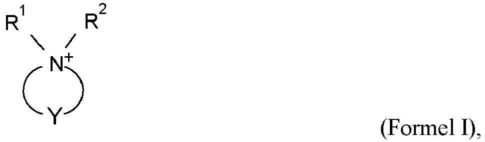 wobei
   Y für ein, ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
   die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
   die N-ständigen Substituenten R¹ und R² untereinander ein Ringsegment X bilden, für das die gleiche oder verschiedene vorstehend für Y gegebene Definition gilt, umgesetzt wird,
die Umsetzung bei Erreichen eines vorgebbaren Umsetzungsgrades, bezogen auf die Gesamtmenge an NCO-Gruppen des mindestens einen organischen Di- und/oder Triisocyanates, durch Zugabe einer unterstöchiometrischen Menge, bezogen auf die molare Menge des Kations der Formel I in Komponente A1, einer Komponente A2, umfassend mindestens eine saure Verbindung, die einen pKₛ-Wert unterhalb 4,0 aufweist und verschieden von HF ist, gestoppt wird
und das erhaltene modifizierte Isocyanat nach optionaler Reinigung mit einer Komponente A3, umfassend mindestens eine saure Verbindung, die einen pKₛ-Wert unterhalb 4,0 aufweist und verschieden von HF ist, versetzt wird, wobei die mindestens eine saure Verbindung in Komponente A2 und A3 verschieden voneinander oder gleich sein können.

Nach einer zweiten Ausführungsform betrifft die Erfindung ein Verfahren zur Herstellung modifizierter Isocyanate gemäß Ausführungsform 1, dadurch gekennzeichnet, dass R¹ und R² im Kation der Formel I unabhängig voneinander für gleiche oder verschiedene Ci-Cs-Alkylsubstituenten oder für gleiche oder verschiedene, ggf. am aromatischen Kern substituierte Benzyl-Reste, bevorzugt für gleiche oder verschiedene C₁-C₆-Alkylsubstituenten, besonders bevorzugt für gleiche oder verschiedene, linear aufgebaute C₁-C₆-Alkylsubstituenten stehen und ganz besonders bevorzugt für Methyl stehen, oder dass R¹ und R² gemeinsam mit dem ladungstragenden Stickstoffatom für ein zu Y gleiches oder von Y verschiedenes Ringsegment X stehen, wobei X ein ggf. weitere Substituenten tragendes C₄-C₆-Alkylenkettensegment ist und optional weitere Substituenten tragen kann.

Nach einer dritten Ausführungsform betrifft die Erfindung ein Verfahren zur Herstellung modifizierter Isocyanate gemäß Ausführungsform 1 oder 2, dadurch gekennzeichnet, dass Y im Kation der Formel I für ein gemeinsam mit dem ladungstragenden Stickstoffatom vier- bis siebengliedriges, ggf. weitere Substituenten tragendes Alkylenkettensegment steht, bevorzugt das Segment Y und/oder das Ringsegment X linear aufgebaut ist.

Nach einer vierten Ausführungsform betrifft die Erfindung ein Verfahren zur Herstellung modifizierter Isocyanate gemäß einer der vorstehenden Ausführungsformen, dadurch gekennzeichnet, dass die Komponente A1 ein Anion enthält, welches ausgewählt ist aus der Gruppe bestehend aus Hydroxid, Alkanoat, Carboxylat, Heterocyclen mit mindestens einem negativ geladenen Stickstoffatom im Ring, Fluorid, Hydrogendifluorid, höhere Polyfluoride, Addukte von mehr als einem Äquivalent HF an Fluorid-Ionen enthaltende Verbindungen und beliebige Mischungen der vorgenannten.

Nach einer fünften Ausführungsform betrifft die Erfindung ein Verfahren zur Herstellung modifizierter Isocyanate gemäß einer der vorstehenden Ausführungsformen, dadurch gekennzeichnet, dass die mindestens eine saure Verbindung in Komponente A1 und/oder A2 einen pK_{S}-Wert unterhalb 2,0 aufweist.

Nach einer sechsten Ausführungsform betrifft die Erfindung ein Verfahren zur Herstellung modifizierter Isocyanate gemäß einer der vorstehenden Ausführungsformen, dadurch gekennzeichnet, dass die Komponente A3 (Stabilisator) in einer molaren Menge zugesetzt wird, die gemeinsam mit Komponente A2 (Stopper) stöchiometrisch, bevorzugt überstöchiometrisch, bezogen auf die Menge des Kations der Formel I in Komponente A1, ist.

Nach einer siebten Ausführungsform betrifft die Erfindung ein Verfahren zur Herstellung modifizierter Isocyanate gemäß einer der vorstehenden Ausführungsformen, dadurch gekennzeichnet, dass die mindestens eine saure Verbindung der Komponente A2 und/oder A3 ausgewählt ist aus der Gruppe umfassend oder bestehend aus Alkan- sowie Aryl-sulfonsäuren wie z.B. Napthalen-mono- sowie disulfonsäuren, Toluolsulfonsäure Dodecylbenzolsulfonsäure, Methansulfonsäure, Phosphorsäure und saure Ester der Phosphorsäure, wie beispielsweise Dibutylphosphat und/oder Monobutylphosphat sowie beliebige Mischungen der vorstehend genannten Verbindungen, bevorzugt aus aromatischen Sulfonsäuren und besonders bevorzugt aus Dodecylbenzolsulfonsäure und Toluolsulfonsäure.

Nach einer achten Ausführungsform betrifft die Erfindung ein Verfahren zur Herstellung modifizierter Isocyanate gemäß einer der vorstehenden Ausführungsformen, dadurch gekennzeichnet, dass das organische Diiisocyanat ausgewählt ist aus der Gruppe umfassend oder bestehend aus PDI, HDI, MPDI, TMDI, NTI, IPDI, IMCI, XDI, H6XDI, MDI, TDI oder NBDI, bevorzugt aus der Gruppe umfassend oder bestehend aus Pentamethylendiisocyanat, Hexamethylendiisocyanat, Isophorondiisocyanat, 1,3- sowie 1,4-Bis(isocyanatomethyl)cyclohexan und/oder Norbornandiisocyanat.

Nach einer neunten Ausführungsform betrifft die Erfindung ein Katalysator-Kit für die Isocyanatmodifizierung, umfassend drei Komponenten A1, A2 und A3, wobei
a) die Komponente A1 mindestens ein Tetramethylammoniumsalz und/oder mindestens ein cyclisches Ammoniumsalz mit einem Kation der Formel I, 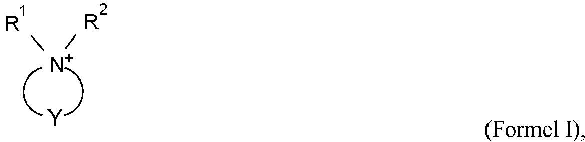 enthält, wobei
   Y für ein, ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
   die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
   die N-ständigen Substituenten R¹ und R² untereinander ein Ringsegment X bilden, für das die gleiche oder verschiedene vorstehend für Y gegebene Definition gilt;
b) die Komponente A2 mindestens eine saure Verbindung umfasst oder daraus besteht, die einen pKₛ-Wert unterhalb 4,0 aufweist und verschieden von HF ist und in einer unterstöchiometrischen Menge, bezogen auf die molare Menge des Kations der Formel I in Komponente A1, eingesetzt wird; und
c) die Komponente A3 mindestens eine saure Verbindung enthält, die einen pKₛ-Wert unterhalb 4,0 aufweist und verschieden von HF ist, wobei die mindestens eine saure Verbindung in Komponente A2 und A3 verschieden voneinander oder gleich sein können.

Nach einer zehnten Ausführungsform betrifft die Erfindung ein Katalysator-Kit für die Isocyanatmodifizierung gemäß Ausführungsform neun, dadurch gekennzeichnet, dass R¹ und R² im Kation der Formel I unabhängig voneinander für gleiche oder verschiedene Ci-Cs-Alkylsubstituenten oder für gleiche oder verschiedene, ggf. am aromatischen Kern substituierte Benzyl-Reste, bevorzugt für gleiche oder verschiedene C₁-C₆-Alkylsubstituenten und besonders bevorzugt für gleiche oder verschiedene, linear aufgebaute C₁-C₆-Alkylsubstituenten stehen oder dass R¹ und R² gemeinsam mit dem ladungstragenden Stickstoffatom für ein zu Y gleiches oder von Y verschiedenes Ringsegment X stehen, wobei X ein ggf. weitere Substituenten tragendes C₄-C₆-Alkylenkettensegment ist und optional weitere Substituenten tragen kann.

Nach einer elften Ausführungsform betrifft die Erfindung ein Katalysator-Kit für die Isocyanatmodifizierung gemäß einer der Ausführungsformen neun oder zehn, dadurch gekennzeichnet, dass Y im Kation der Formel I für ein gemeinsam mit dem ladungstragenden Stickstoffatom vier- bis sieben-gliedriges, ggf. weitere Substituenten tragendes Alkylenkettensegment steht, bevorzugt ist das Segment Y und/oder das Ringsegment X linear aufgebaut.

Nach einer zwölften Ausführungsform betrifft die Erfindung ein Katalysator-Kit für die Isocyanatmodifizierung gemäß einer der Ausführungsform neun bis elf, dadurch gekennzeichnet, dass die Komponente A1 ein Anion enthält, welches ausgewählt ist aus der Gruppe bestehend aus Hydroxid, Alkanoat, Carboxylat, Heterocyclen mit mindestens einem negativ geladenen Stickstoffatom im Ring, Fluorid, Hydrogendifluorid, höhere Polyfluoride, Addukte von mehr als einem Äquivalent HF an Fluorid-Ionen enthaltende Verbindungen und beliebige Mischungen der vorgenannten.

Nach einer dreizehnten Ausführungsform betrifft die Erfindung ein Katalysator-Kit für die Isocyanatmodifizierung gemäß einer der Ausführungsform neun bis zwölf, dadurch gekennzeichnet, dass die mindestens eine saure Verbindung in Komponente A1 und/oder A2 einen pK_{S}-Wert unterhalb 2,0 aufweist.

Nach einer vierzehnten Ausführungsform betrifft die Erfindung ein Katalysator-Kit für die Isocyanatmodifizierung gemäß einer der Ausführungsform neun bis dreizehn, dadurch gekennzeichnet, dass die Komponente A3 (Stabilisator) in einer molaren Menge zugesetzt wird, die gemeinsam mit Komponente A2 (Stopper) stöchiometrisch, bevorzugt überstöchiometrisch, bezogen auf die Menge des Kations der Formel I in Komponente A 1, ist.

Nach einer fünfzehnten Ausführungsform betrifft die Erfindung ein Katalysator-Kit für die Isocyanatmodifizierung gemäß einer der Ausführungsformen neun bis vierzehn, dadurch gekennzeichnet, dass die mindestens eine saure Verbindung der Komponente A2 und/oder A3 ausgewählt ist aus der Gruppe umfassend oder bestehend aus Alkan- sowie Aryl-sulfonsäuren wie z.B. Napthalen-mono- sowie disulfonsäuren, Toluolsulfonsäure, Dodecylbenzolsulfonsäure, Methansulfonsäure, Phosphorsäure und saure Ester der Phosphorsäure, wie beispielsweise Dibutylphosphat und/oder Monobutylphosphat sowie beliebige Mischungen der vorstehend genannten Verbindungen, bevorzugt aus aromatischen Sulfonsäuren und besonders bevorzugt aus Dodecylbenzolsulfonsäure und Toluolsulfonsäure.

Nach einer sechzehnten Ausführungsform betrifft die Erfindung eine Verwendung mindestens des Katalysator-Kits nach einer der Ausführungsformen neun bis fünfzehn in der Isocyanatmodifizierung zur Verhinderung von Trübungen im modifizierten Isocyanat.

Nach einer siebzehnten Ausführungsform betrifft die Erfindung ein modifiziertes Isocyanat, erhältlich oder hergestellt, bevorzugt unmittelbar erhältlich durch ein Verfahren nach einer der Ausführungsformen eins bis acht.

Nach einer achtzehnten Ausführungsform betrifft die Erfindung ein Ein-Komponenten-System, enthaltend ein modifiziertes Isocyanat nach Ausführungsform siebzehn, bei dem die NCO-Gruppen blockiert sind, oder ein Zwei-Komponenten-System, enthaltend eine Komponente 1), umfassend mindestens ein modifiziertes Isocyanat nach Ausführungsform siebzehn, und eine Komponente 2), umfassend mindestens eine gegenüber NCO-Gruppen reaktive Verbindung.

Nach einer neunzehnten Ausführungsform betrifft die Erfindung eine Beschichtung, erhältlich oder hergestellt durch Aufbringen eines Ein- oder Zwei-Komponenten-Systems nach Ausführungsform achtzehn auf ein Substrat und Aushärten, gegebenenfalls unter Wärmeeinwirkung und/oder in Anwesenheit eines Katalysators.

Nach einer zwanzigsten Ausführungsform betrifft die Erfindung ein Verbundbauteil, umfassend einen Werkstoff, der zumindest anteilig mit einer Beschichtung nach Ausführungsform neunzehn verbunden ist.

Die nachfolgenden Vergleichsbeispiele und Beispiele sollen die Erfindung näher erläutern, ohne sie jedoch einzuschränken.

### Beispiele:

Alle Prozentangaben sind, soweit nicht anders vermerkt, als Gewichtsprozent zu verstehen.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brücker an ca. 5 %igen (¹H-NMR) bzw. ca. 50 %igen (¹³C-NMR) Proben in trockenem C₆D₆ bei einer Frequenz von 400 bzw. 700 MHz (¹H-NMR) oder 100 bzw. 176 MHz (¹³C-NMR). Als Referenz für die ppm-Skale wurden geringe Mengen von Tetramethylsilan im Lösungsmittel mit 0 ppm ¹H-NMR-chem. Verschiebung herangezogen. Alternativ wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP 896 009 A1).

Die dynamischen Viskositäten wurden bei 23 °C mit dem Viskosimeter VT 550 der Fa. Haake nach der DIN EN ISO 3219:1994-10 bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Ermittlung des NCO-Gehaltes erfolgte durch Titration gemäß DIN EN ISO 11909:2007-05.

Die Bestimmung der Restmonomergehalte erfolgte gaschromatographisch nach der DIN EN ISO 10283:2007-11 mit internem Standard.

Alle Reaktionen wurden, wenn nicht anders angegeben, unter einer Stickstoffatmosphäre durchgeführt.

Die verwendeten Diisocyanate sind Produkte der Covestro AG, D-51365 Leverkusen, alle anderen kommerziell zugänglichen Chemikalien wurden von der Fa. Aldrich, D-82018 Taufkirchen, bezogen.

Die nicht kommerziell erhältlichen Edukte wurden nach literaturbekannten Methoden gewonnen.

### Beispiel 1: Katalysatorherstellung (nicht erfindungsgemäß)

In einem 2 1 Vierhalskolben mit Rührer und Intensivkühler wurden 61,7 g (1,1 mol) KOH, 500 ml Wasser (entionisiert) und 139,7 g (1,1 mol) 1,4-Dichlorbutan gemeinsam vorgelegt und unter Rühren auf ca. 100-110°C Ölbadtemperatur erhitzt (schwacher Rückfluß).

Nach Erreichen der o.g. Innentemperatur wurden 85,2 g (1 mol) Piperidin so zügig zugegeben, dass nicht zu heftiger Rückfluss eintrat.

Bereits kurz nach Beginn der Piperidinzugabe setzte Feststoffabscheidung ein. Das Reaktionsgemisch war von Beginn an heterogen (anfangs flüssig-flüssig, dann dreiphasig flüssig-flüssig-fest, dann fest-flüssig), enthielt aber immer genügend flüssige Phase für eine effiziente Durchmischung des Großteils der Reaktionsmasse.

Nach vollständiger Piperidinzugabe wurde zwei Stunden bei 110 - 120°C Ölbadtemperatur nachgerührt. Der Rückfluss ließ in diesem Zeitraum merklich nach.

Anschließend wurden in die noch heiße Mischung ca. 150 g Methanol dosiert und so lange am Rückfluss gekocht, bis eine feinteilige Suspension entstanden war. Anschließend wurde auf ca. 40 °C abgekühlt, es wurden ca. 87 g (1,5 mol) festes KF dosiert und mit hinreichend viel Methanol (250 - 300 g, genaue Menge unkritisch) beaufschlagt, um gute Rührbarkeit zu gewährleisten.

Anschließend wurde bei 40°C unter gelegentlicher argentometrischer Chloridkontrolle der vorher filtrierten, flüssigen Phase gerührt und anschließend filtriert. Der Filterrückstand wurde portionsweise mit summarisch ca. 400 g auf ca. 40-50°C vorgewärmtem 2-Ethylhexanol (2-EH) gewaschen und die vereinigten Filtrate unter Rühren und Kühlung mit 50 g (1 mol HF) 40%iger wässriger Flusssäure so versetzt, dass die Innentemperatur nicht über 40°C anstieg.

Nach Vollständiger HF-Zugabe wurde eine Stunde zum Rückfluss erhitzt und anschließend auf Destillatabnahme gestellt. Nachdem bei Normaldruck und 100°C Badtemperatur kein Destillat mehr überging, wurde der Druck stufenweise auf 50 mbar reduziert, die Sumpftemperatur stufenweise auf maximal 150°C gesteigert und das anfallende Destillat diskontinuierlich ausgeschleust. Zum Ende stand wasserfreies Destillat (2-EH) am Kopf an.

Nach Abkühlen wurde durch 2-EH-Zugabe auf Sollkonzentration (20 %) eingestellt und von geringen Mengen unlöslicher Bestandteile abfiltriert. Der gesamt-Fluoridgehalt dieser klaren, leicht gelben Lösung (ionensensitive Elektrode; hierbei werden F ex HF und Fluorid aus dem Halogenaustausch summarisch als Fluorid erfasst) betrug 4,3%. Das aus der HF-Zugabe herrührende F wurde acidimetrisch (als H⁺ ex HF) mit 0,1 N NaOH gegen Phenolphthalein titriert und zu 2,1% gefunden, der (argentometrisch bestimmte) Rest-Chloridgehalt belief sich auf 0,15 %.

Die anderen in Tabelle 1 aufgeführten Katalysatoren wurden entsprechend aus dem primär gebildeten Chlorid durch Anionenaustausch (optional gefolgt von HF-Zugabe für die Synthese der Di/Polyfluoride) synthetisiert. Die Monocyclischen Ammoniumsalze 5-7 wurden auf Basis kommerziell zugänglicher N,N-Dimethylammonium chloride ebenfalls wie vorstehend dargestellt durch Anionenaustausch (optional gefolgt von HF-Zugabe für die Synthese der Di/Polyfluoride) synthetisiert.

**Tabelle 1: Übersicht der hergestellten Katalysatoren (die Katalysatorkonzentration bezieht sich auf den Wirkstoff (Kation und Anion))**

| Katalysator | Kation | Anion | Konzentration [%] |
|---|---|---|---|
| 1 5 -Azoniaspiro [4.4] nonanium hydroxid | 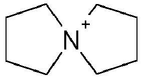 | OH⁻ | 1 |
| 2 5-Azoniaspiro[4.5] decanium hydrogendifluorid | 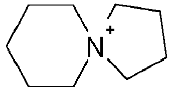 | [HF₂]⁻ | 20 |
| 3 6-Azoniaspiro[5.5] undecanium pivalat | 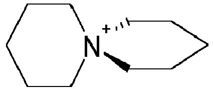 | (CH₃)₃CC(O)⁻ | 10 |
| 4 6-Azoniaspiro[5.5] undecanium hydrogendifluorid | 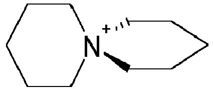 | [HF₂]⁻ | 20 |
| 5 1,1-Dimethylpyrrolidin-1-ium hydroxid | 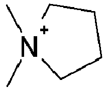 | OH⁻ | 1 |
| 6 1,1-Dimethylpyrrolidin-1-ium hydrogendifluorid | 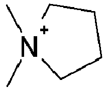 | [HF₂]⁻ | 10 |
| 7 1,1-Dimethylpiperidin-1-ium pivalat | 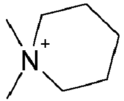 | (CH₃)₃CC(O)O⁻ | 10 |

### Beispielserie 2: nicht erfindungsgemäß, Verfahrensweise gemäß Beispielserien 2-1 bis 2-4 der EP 3107948 sowie Beispielserien 2-5 bis 2-7 der EP 3337836

Die optimale Katalysatorkonzentration für die Diisocyanat-Trimerisierung wurde in orientierenden Vorversuchen bei 60 °C ermittelt und die Konzentration der Katalysatorlösung durch Verdünnen mit 2-EH so eingestellt, dass keine bzw. nur eine vernachlässigbar geringe Gelteilchenbildung bei der Zugabe der Katalysatorlösung zum gerührten HDI zu beobachten war. Anschließend wurden die Reihenversuche mit Destillat-Recyclisierung im 1 kg Maßstab wie nachstehend beschrieben durchgeführt.

In einem doppelwandigen, durch einen externen Kreislauf auf die jeweils gewünschte Starttemperatur temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (< 1 mbar) von gelösten Gasen befreit. Nach Belüften mit Stickstoff wurde die in Tabelle 2 angegebene Katalysator-art und -menge in der in Tabelle 2 aufgeführten Konzentration so dosiert, dass die Reaktion im Temperaturbereich zwischen ca. 60 und 65°C gefahren werden konnte. Nachdem ca. 1 mol NCO Gruppen umgesetzt waren, indiziert durch Erreichen eines NCO-Gehaltes um 45,8 %, wurde der Katalysator durch Zugabe einer zum Katalysator äquivalenten Menge des in Tabelle 2 angegebenen Stoppers deaktiviert, weitere 30 min bei Reaktionstemperatur nachgerührt und anschließend aufgearbeitet.

Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 +/- 0,04 mbar, VV-Temperatur: 120 °C, HV-Temp.: 140 °C), wobei nicht umgesetztes Monomer als Destillat und das monomerenarme Polyisocyanatharz als Sumpfprodukt separiert wurden (Startdurchlauf). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde erneut katalysiert und verfahren wie eingangs beschrieben. Diese Verfahrensweise wurde mehrmals wiederholt (Versuche A, B, C, usw.). Nach mehreren Recyclierschritten war zu beobachten, dass die Katalysatormenge, die zum Erreichen des angestrebten Umsatzes nötig war, beim ersten Versuch jeder Serie zunächst etwas höher war, als beim Folgeversuch, aber danach sukzessive anstieg. Weiterhin war, insbesondere bei den Produkten der "späteren" Recyclierschritte (hier Versuche -D ff.) und oft erst nach mehrwöchiger Lagerung und/oder nach Zugabe typischer Lacklösemittel wie Xylol oder Solventnaphtha, eine langsam einsetzende Trübung bei den Polyisocyanatharzen, mitunter begleitet von einem langsamen Anstieg des Restmonomerengehates, insbesondere bei der Lagerung bei höherer Temperatur, zu verzeichnen. Die nach Filtration erhaltenen Niederschläge erwiesen sich nach Ausweis kombinierter analytischer Methoden im Wesentlichen als die Ammonium Chloride (molar ca. 2/3) sowie Bromide (molar ca. 1/3) der eingesetzten Katalysatoren.

Die Ergebnisse sind Tabelle 2 zu entnehmen.

**Tabelle 2: Durchgeführte Isocyanatmodifizierungen (Vergleichsbeispiele)**

| Beispiel-Nr. 2-... | Katalysator lt. Tab. 1 | Katalysatorlsg. [g] | Stopper^{∗)} |
|---|---|---|---|
| -1-A | 1 | 7,9 | 1 |
| -1-B | 1 | 6,1 | 1 |
| -1-C | 1 | 6,2 | 1 |
| -1-D | 1 | 6,4 | 1 |
| -1-E | 1 | 6,5 | 1 |
| -1-F | 1 | 7,1 | 1 |
| -2-A | 2 | 0,28 | 2 |
| -2-B | 2 | 0,20 | 2 |
| -2-C | 2 | 0,21 | 2 |
| -2-D | 2 | 0,25 | 2 |
| -2-E | 2 | 0,31 | 2 |
| -2-F | 2 | 0,45 | 2 |
| -3-A | 3 | 1,92 | 1 |
| -3-B | 3 | 1,70 | 1 |
| -3-C | 3 | 1,72 | 1 |
| -3-D | 3 | 1,85 | 1 |
| -3-E | 3 | 2,01 | 1 |
| -3-F | 3 | 2,94 | 1 |
| -4-A | 4 | 0,58 | 3 |
| -4-B | 4 | 0,52 | 3 |
| -4-C | 4 | 0,61 | 3 |
| -4-D | 4 | 0,68 | 3 |
| -4-E | 4 | 0,72 | 3 |
| -4-F | 4 | 0,89 | 3 |
| -5-A | 5 | 0,86 | 3 |
| -5-B | 5 | 0,90 | 3 |
| -5-C | 5 | 0,65 | 3 |
| -5-D | 5 | 1,24 | 3 |
| -5-E | 5 | 1,56 | 3 |
| -5-F | 5 | 1,80 | 3 |
| -6-A | 6 | 1,45 | 2 |
| -6-B | 6 | 0,92 | 2 |
| -6-C | 6 | 1,01 | 2 |
| -6-D | 6 | 1,25 | 2 |
| -6-E | 6 | 1,35 | 2 |
| -6-F | 6 | 1,52 | 2 |
| -7-A | 7 | 4,30 | 1 |
| -7-B | 7 | 3,98 | 1 |
| -7-C | 7 | 4,10 | 1 |
| -7-D | 7 | 4,69 | 1 |
| -7-E | 7 | 5,10 | 1 |
| -7-F | 7 | 5,26 | 1 |

| | | | |
|---|---|---|---|
| ^{∗)} Stopper: 1: Di-n-butylphosphat, 2: Toluolsulfonsäure, 40%ig in 2-PrOH, 3: Dodecylbenzolsulfonsäure, 70%ig in 2-PrOH | | | |

### Beispielserie 3: erfindungsgemäß

Es wurde verfahren wie in Beispielserie 2 angegeben, mit dem Unterschied, das nach Erreichen des Ziel-NCO-Gehaltes (um 45,8 %), der Katalysator durch Zugabe einer zum Katalysator unter-stöchiometrischen Menge (40 - 60 mol-%, auf eingesetzten Katalysator bezogen) des in Tabelle 3 angegebenen Stoppers deaktiviert, weitere 30 min bei Reaktionstemperatur nachgerührt wurde, um sicherzustellen, dass die Reaktion wirkungsvoll unterbunden wurde und anschließend aufgearbeitet wurde wie in Beispielserie 2 angegeben.

Das nach jedem Umlauf isolierte Polyisocyanatharz wurde separiert und im Anschluß mit der zu 110% der stöchiometrisch auf eingesetzten Katalysator bezogenen Stoppermenge fehlenden Menge nachstabilisiert, indem nach Zugabe des Stabilisators bei 60°C eine Stunde gerührt wurde. Auch nach mehreren Recyclierschritten war kein Anstieg in der zum Erreichen des Sollumsatzes nötigen Katalysatormenge im jeweiligen Folgeversuch zu verzeichnen. Weiterhin war bei keinem der erhaltenen Produkte unter den in der Vergleichsbeispielserie 2 genannten Bedingungen eine verzögert einsetzende Trübung bei den Polyisocyanatharzen oder ein Anstieg des Restmonomerengehaltes zu verzeichnen.

Die Ergebnisse sind Tabelle 3 zu entnehmen.

**Tabelle 3: Durchgeführte Isocyanatmodifizierungen (erfindungsgemäße Beispiele)**

| Beispiel-Nr. 2-... | Katalysator lt. Tab. 1 | Katalysatorlsg. [g] | Stopper^{∗)} |
|---|---|---|---|
| -1-A | 1 | 7,8 | 1 |
| -1-B | 1 | 6,0 | 1 |
| -1-C | 1 | 5,9 | 1 |
| -1-D | 1 | 6,0 | 1 |
| -1-E | 1 | 6,0 | 1 |
| -1-F | 1 | 6,0 | 1 |
| -2-A | 2 | 0,3 | 2 |
| -2-B | 2 | 0,2 | 2 |
| -2-C | 2 | 0,2 | 2 |
| -2-D | 2 | 0,2 | 2 |
| -2-E | 2 | 0,2 | 2 |
| -2-F | 2 | 0,2 | 2 |
| -3-A | 3 | 1,9 | 1 |
| -3-B | 3 | 1,7 | 1 |
| -3-C | 3 | 1,7 | 1 |
| -3-D | 3 | 1,6 | 1 |
| -3-E | 3 | 1,6 | 1 |
| -3-F | 3 | 1,6 | 1 |
| -4-A | 4 | 0,61 | 3 |
| -4-B | 4 | 0,50 | 3 |
| -4-C | 4 | 0,52 | 3 |
| -4-D | 4 | 0,51 | 3 |
| -4-E | 4 | 0,50 | 3 |
| -4-F | 4 | 0,50 | 3 |
| -5-A | 5 | 0,91 | 3 |
| -5-B | 5 | 0,85 | 3 |
| -5-C | 5 | 0,80 | 3 |
| -5-D | 5 | 0,81 | 3 |
| -5-E | 5 | 0,80 | 3 |
| -5-F | 5 | 0,81 | 3 |
| -6-A | 6 | 1,45 | 2 |
| -6-B | 6 | 1,0 | 2 |
| -6-C | 6 | 1,0 | 2 |
| -6-D | 6 | 1,0 | 2 |
| -6-E | 6 | 1,0 | 2 |
| -6-F | 6 | 1,0 | 2 |
| -7-A | 7 | 4,3 | 1 |
| -7-B | 7 | 3,9 | 1 |
| -7-C | 7 | 3,85 | 1 |
| -7-D | 7 | 3,8 | 1 |
| -7-E | 7 | 3,8 | 1 |
| -7-F | 7 | 3,75 | 1 |

| | | | |
|---|---|---|---|
| ^{∗)} Stopper/Stabilisator: 1: Di-n-butylphosphat, 2: Toluolsulfonsäure, 40%ig in 2-PrOH, 3: Dodecylbenzolsulfonsäure, 70%ig in 2-PrOH | | | |

### Beispielserie 4: erfindungsgemäß

Es wurde verfahren wie in Beispielserie 3 angegeben, mit dem Unterschied, das statt HDI PDI (1,5-Pentamethylendiisocyanat) verwendet wurde und nach Erreichen des Ziel-NCO-Gehaltes (54,9 bis 55,1 %), der Katalysator wie in Beispielserie 3 beschrieben durch Zugabe einer zum Katalysator unter-stöchiometrischen Menge (40 - 60 mol-%, auf eingesetzten Katalysator bezogen) des in Tabelle 4 angegebenen Stoppers deaktiviert, weitere 30 min bei Reaktionstemperatur nachgerührt wurde, um sicherzustellen, dass die Reaktion wirkungsvoll unterbunden wurde und anschließend aufgearbeitet wurde wie in Beispielserien 2 bzw. 3 angegeben.

Das nach jedem Umlauf isolierte Polyisocyanatharz wurde separiert und im Anschluß mit der zu 110% der stöchiometrisch auf eingesetzten Katalysator bezogenen Stoppermenge fehlenden Menge nachstabilisiert, indem nach Zugabe des Stabilisators bei 60°C eine Stunde gerührt wurde. Auch nach mehreren Recyclierschritten war kein Anstieg in der zum Erreichen des Sollumsatzes nötigen Katalysatormenge im jeweiligen Folgeversuch zu verzeichnen. Weiterhin war bei keinem der erhaltenen Produkte unter den in der Vergleichsbeispielserie 2 genannten Bedingungen eine verzögert einsetzende Trübung bei den Polyisocyanatharzen oder ein Anstieg des Restmonomerengehaltes zu verzeichnen.

Die Ergebnisse sind Tabelle 4 zu entnehmen.

**Tabelle 4: Durchgeführte Isocyanatmodifizierungen (erfindungsgemäße Beispiele)**

| Beispiel-Nr. 4-... | Katalysator lt. Tab. 1 | Katalysatorlsg. [g] | Stopper^{∗)} |
|---|---|---|---|
| -1-A | 1 | 8,2 | 1 |
| -1-B | 1 | 8,0 | 1 |
| -1-C | 1 | 8,0 | 1 |
| -1-D | 1 | 7,9 | 1 |
| -1-E | 1 | 7,8 | 1 |
| -1-F | 1 | 7,7 | 1 |
| -2-A | 2 | 0,5 | 2 |
| -2-B | 2 | 0,4 | 2 |
| -2-C | 2 | 0,3 | 2 |
| -2-D | 2 | 0,3 | 2 |
| -2-E | 2 | 0,3 | 2 |
| -2-F | 2 | 0,3 | 2 |
| -3-A | 3 | 2,5 | 1 |
| -3-B | 3 | 2,4 | 1 |
| -3-C | 3 | 2,4 | 1 |
| -3-D | 3 | 2,3 | 1 |
| -3-E | 3 | 2,3 | 1 |
| -3-F | 3 | 2,2 | 1 |
| -4-A | 4 | 0,8 | 3 |
| -4-B | 4 | 0,7 | 3 |
| -4-C | 4 | 0,7 | 3 |
| -4-D | 4 | 0,6 | 3 |
| -4-E | 4 | 0,5 | 3 |
| -4-F | 4 | 0,4 | 3 |
| -5-A | 5 | 1,2 | 3 |
| -5-B | 5 | 1,1 | 3 |
| -5-C | 5 | 1,1 | 3 |
| -5-D | 5 | 1,0 | 3 |
| -5-E | 5 | 0,9 | 3 |
| -5-F | 5 | 0,9 | 3 |
| -6-A | 6 | 1,7 | 2 |
| -6-B | 6 | 1,6 | 2 |
| -6-C | 6 | 1,5 | 2 |
| -6-D | 6 | 1,5 | 2 |
| -6-E | 6 | 1,4 | 2 |
| -6-F | 6 | 1,4 | 2 |
| -7-A | 7 | 4,5 | 1 |
| -7-B | 7 | 4,2 | 1 |
| -7-C | 7 | 4,1 | 1 |
| -7-D | 7 | 4,1 | 1 |
| -7-E | 7 | 4,0 | 1 |
| -7-F | 7 | 4,0 | 1 |

| | | | |
|---|---|---|---|
| ^{∗)} Stopper/Stabilisator: 1: Di-n-butylphosphat, 2: Toluolsulfonsäure, 40%ig in 2-PrOH, 3: Dodecylbenzolsulfonsäure, 70%ig in 2-PrOH | | | |

## Patentansprüche

1. Verfahren zur Herstellung modifizierter Isocyanate, bei dem mindestens ein organisches Di- und/oder Triisocyanat in zumindest zeitweiliger Anwesenheit einer Komponente A1 umfassend mindestens ein Tetramethylammoniumsalz und/oder mindestens ein cyclisches Ammoniumsalz mit einem Kation der Formel I, 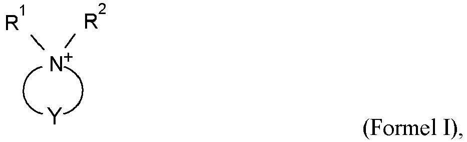 wobei
Y für ein, ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
die N-ständigen Substituenten R¹ und R² entweder unabhängig voneinander für gleiche oder ver¬schiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
die N-ständigen Substituenten R¹ und R² untereinander ein Ringsegment X bilden, für das die gleiche oder verschiedene vorstehend für Y gegebene Definition gilt, umgesetzt wird,
die Umsetzung bei Erreichen eines vorgebbaren Umsetzungsgrades, bezogen auf die Gesamtmenge an NCO-Gruppen des mindestens einen organischen Di- und/oder Triisocyanates, durch Zugabe einer unterstöchiometrischen Menge, bezogen auf die molare Menge des Kations der Formel I in Komponente A1, einer Komponente A2, umfassend mindestens eine saure Verbindung, die einen pKₛ-Wert unterhalb 4,0 aufweist und verschieden von HF ist, gestoppt wird
und das erhaltene modifizierte Isocyanat nach optionaler Reinigung mit einer Komponente A3, umfassend mindestens eine saure Verbindung, die einen pKₛ-Wert unterhalb 4,0 aufweist und verschieden von HF ist, versetzt wird,
wobei die mindestens eine saure Verbindung in Komponente A2 und A3 verschieden voneinander oder gleich sein können.

2. Das Verfahren nach Anspruch 1, **dadurch gekennzeichnet, dass** R¹ und R² im Kation der Formel I unabhängig voneinander für gleiche oder verschiedene C₁-C₈-Alkylsubstituenten oder für gleiche oder verschiedene, ggf. am aromatischen Kern substituierte Benzyl-Reste, bevorzugt für gleiche oder verschiedene C₁-C₆-Alkylsubstituenten, besonders bevorzugt für gleiche oder verschiedene, linear aufgebaute C₁-C₆-Alkylsubstituenten stehen und ganz besonders bevorzugt für Methyl stehen, oder dass R¹ und R² gemeinsam mit dem ladungstragenden Stickstoffatom für ein zu Y gleiches oder von Y verschiedenes Ringsegment X stehen, wobei X ein ggf. weitere Substituenten tragendes C₄-C₆-Alkylenkettensegment ist und optional weitere Substituenten tragen kann.

3. Das Verfahren nach Anspruch 1 oder 2, **dadurch gekennzeichnet, dass** Y im Kation der Formel I für ein gemeinsam mit dem ladungstragenden Stickstoffatom vier - bis siebengliedriges, ggf. weitere Substituenten tragendes Alkylenkettensegment steht, bevorzugt das Segment Y und/oder das Ringsegment X linear aufgebaut ist.

4. Das Verfahren nach einem der vorstehenden Ansprüche, **dadurch gekennzeichnet, dass** die Komponente A1 ein Anion enthält, welches ausgewählt ist aus der Gruppe bestehend aus Hydroxid, Alkanoat, Carboxylat, Heterocyclen mit mindestens einem negativ geladenen Stickstoffatom im Ring, Fluorid, Hydrogendifluorid, höhere Polyfluoride, Addukte von mehr als einem Äquivalent HF an Fluorid-Ionen enthaltende Verbindungen und beliebige Mischungen der vorgenannten.

5. Das Verfahren nach einem der vorstehenden Ansprüche, **dadurch gekennzeichnet, dass** die mindestens eine saure Verbindung in Komponente A1 und/oder A2 einen pK_{S}-Wert von unterhalb 2,0 aufweist.

6. Das Verfahren nach einem der vorstehenden Ansprüche, **dadurch gekennzeichnet, dass** die Komponente A3 (Stabilisator) in einer molaren Menge zugesetzt wird, die gemeinsam mit Komponente A2 (Stopper) stöchiometrisch, bevorzugt überstöchiometrisch, bezogen auf die Menge des Kations der Formel I in Komponente A1, ist.

7. Das Verfahren nach einem der vorstehenden Ansprüche, **dadurch gekennzeichnet, dass** die mindestens eine saure Verbindung der Komponente A2 und/oder A3 ausgewählt ist aus der Gruppe umfassend oder bestehend aus Alkan- sowie Aryl-sulfonsäuren wie z.B. Napthalen-mono- sowie disulfonsäuren, Toluolsulfonsäure Dodecylbenzolsulfonsäure, Methansulfonsäure, Phosphorsäure und saure Ester der Phosphorsäure, wie beispielsweise Dibutylphosphat und/oder Monobutylphosphat sowie beliebige Mischungen der vorstehend genannten Verbindungen, bevorzugt aus aromatischen Sulfonsäuren und besonders bevorzugt aus Dodecylbenzolsulfonsäure und Toluolsulfonsäure.

8. Das Verfahren nach einem der vorstehenden Ansprüche, **dadurch gekennzeichnet, dass** das organische Diiisocyanat ausgewählt ist aus der Gruppe umfassend oder bestehend aus PDI, HDI, MPDI, TMDI, NTI, IPDI, IMCI, XDI, H6XDI, MDI, TDI oder NBDI, bevorzugt aus der Gruppe umfassend oder bestehend aus Pentamethylendiisocyanat, Hexamethylendiisocyanat, Isophorondiisocyanat, 1,3- sowie 1,4-Bis(isocyanatomethyl)cyclohexan und/oder Norbornandiisocyanat.

9. Katalysator-Kit für die Isocyanatmodifizierung, umfassend drei separate Komponenten A1, A2 und A3, wobei
a) die Komponente A1 mindestens ein Tetramethylammoniumsalz und/oder mindestens ein cyclisches Ammoniumsalz mit einem Kation der Formel I, 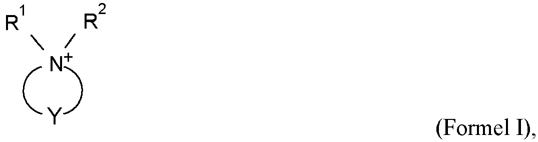 enthält, wobei
Y für ein, ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
die N-ständigen Substituenten R¹ und R² untereinander ein Ringsegment X bilden, für das die gleiche oder verschiedene vorstehend für Y gegebene Definition gilt;
b) die Komponente A2 in einer unterstöchiometrischen Menge, bezogen auf die molare Menge des Kations der Formel I in Komponente A1, enthält und mindestens eine saure Verbindung umfasst oder daraus besteht, die einen pKₛ-Wert unterhalb 4,0 aufweist und verschieden von HF ist;
c) die Komponente A3 mindestens eine saure Verbindung enthält, die einen pKₛ-Wert unterhalb 4,0 aufweist und verschieden von HF ist, und
wobei die mindestens eine saure Verbindung in Komponente A2 und A3 verschieden oder gleich voneinander sein können.

10. Katalysator-Kit nach Anspruch 9, **dadurch gekennzeichnet, dass** die mindestens eine saure Verbindung der Komponente A2 und/oder A3 ausgewählt ist aus der Gruppe umfassend oder bestehend aus Alkan- sowie Aryl-sulfonsäuren wie z.B. Napthalen-mono- sowie disulfonsäuren, Toluolsulfonsäure Dodecylbenzolsulfonsäure, Methansulfonsäure, Phosphorsäure und saure Ester der Phosphorsäure, wie beispielsweise Dibutylphosphat und/oder Monobutylphosphat sowie beliebige Mischungen der vorstehend genannten Verbindungen, bevorzugt aus aromatischen Sulfonsäuren und besonders bevorzugt aus Dodecylbenzolsulfonsäure und Toluolsulfonsäure.

11. Verwendung mindestens des Katalysator-Kits nach Anspruch 9 oder 10 in der Isocyanatmodifizierung zur Verhinderung von Trübungen im modifizierten Isocyanat.

12. Modifiziertes Isocyanat, erhältlich oder hergestellt, bevorzugt unmittelbar erhältlich, durch ein Verfahren nach einem der Ansprüche 1 bis 8.

13. Ein-Komponenten-System, enthaltend ein modifiziertes Isocyanat nach Anspruch 12, bei dem die NCO-Gruppen blockiert sind, oder Zwei-Komponenten-System, enthaltend eine Komponente 1), umfassend mindestens ein modifiziertes Isocyanat nach Anspruch 12, und eine Komponente 2), umfassend mindestens eine gegenüber NCO-Gruppen reaktive Verbindung.

14. Beschichtung, erhältlich oder hergestellt durch Aufbringen eines Ein- oder Zwei-Komponenten-Systems nach Anspruch 13 auf ein Substrat und Aushärten, gegebenenfalls unter Wärmeeinwirkung und/oder in Anwesenheit eines Katalysators.

15. Verbundbauteil, umfassend einen Werkstoff, der zumindest anteilig mit einer Beschichtung nach Anspruch 14 verbunden ist.
